Europäisches Patentamt

European Patent Office

Office européen des brevets

(11) Numéro de publication : **0 388 787 B1**

(12) **FASCICULE DE BREVET EUROPEEN**

(45) Date de publication du fascicule du brevet :
**19.11.92 Bulletin 92/47**

(51) Int. Cl.⁵ : **F04B 43/12, A61M 5/142**

(21) Numéro de dépôt : **90104883.5**

(22) Date de dépôt : **15.03.90**

(54) **Pompe péristaltique miniature.**

(30) Priorité : **24.03.89 FR 8904044**

(43) Date de publication de la demande :
**26.09.90 Bulletin 90/39**

(45) Mention de la délivrance du brevet :
**19.11.92 Bulletin 92/47**

(84) Etats contractants désignés :
**CH DE GB IT LI NL SE**

(56) Documents cités :
**FR-A- 2 166 467**
**FR-A- 2 479 692**
**GB-A- 2 138 511**
**US-A- 3 742 697**
**LIFE SUPPORT SYSTEMS, vol. 1, janvier/mars
1983, pages 23-38, European Society for Artificial Organs, Eastbourne, EastSussex, GB; K.
PRESTELE et al.: "Development of remotely
controlled implantable devices for programmed insulin infusion"**

(73) Titulaire : **ASULAB S.A.
Faubourg du Lac 6
CH-2501 Bienne (CH)**

(72) Inventeur : **Gagnebin, Eric
Fleur-de-Lys 23
CH-2074 Marin (CH)**
Inventeur : **Meyrat, Clément
Route de Bâle 9
CH-2525 Le Landeron (CH)**
Inventeur : **Dubois, Antoine
Le Chauffaud
F-25130 Villers-le-Lac (FR)**

(74) Mandataire : **de Raemy, Jacques et al
ICB Ingénieurs Conseils en Brevets SA
Passage Max. Meuron 6
CH-2001 Neuchâtel (CH)**

Jouve, 18, rue Saint-Denis, 75001 PARIS

## Description

La présente invention est relative à une pompe péristaltique miniature portable notamment sur le corps humain pour l'injection lente et continue de préparations médicamenteuses à l'état acqueux, comportant une pompe équipée d'un rotor porté par un arbre autour duquel sont également répartis des galets animés d'un mouvement de révolution roulant le long d'au moins un tuyau flexible en le comprimant localement contre au moins un presseur entourant ledit tuyau selon un arc déterminé de manière à permettre l'aspiration et le refoulement de ladite préparation médicamenteuse, ledit arbre étant muni d'un élément de couplage, un moteur pas à pas entraînant un rouage démultiplicateur portant en fin de chaîne une prise de force, un circuit de commande pour fournir au moteur des impulsions d'alimentation issues d'une base de temps suivie d'un diviseur de fréquence et une pile d'alimentation.

Tant à l'usage de la recherche qu'à l'emploi thérapeutique, on connaît l'utilisation des pompes péristaltiques qui permettent le dosage précis de liquide à administrer, par exemple à un patient, sous forme de perfusion. On trouvera dans les documents US-A-4 715 786 et WO 88/10 372 des descriptions de pompes péristaltiques dont le principe est bien connu et qui consiste très généralement à utiliser un tube élastiquement déformable, à l'écraser localement contre un carter au moyen d'un rotor équipé de galets tournants et à aspirer ainsi puis à refouler le liquide contenu dans le tube en provenance d'un réservoir. En variant la vitesse de rotation du rotor, on peut modifier le débit de la pompe. Ce débit peut même être programmé dans le temps, ceci en fonction des exigences imposées par la maladie à soigner ou par l'expérience que l'on se propose de conduire.

Les pompes péristaltiques équipées d'un seul tube ou tuyau flexible présentent le défaut de débiter de manière sinusoïdale, donc irrégulièrement le liquide qu'elles sont chargées de véhiculer. Pour y remédier, on a proposé l'utilisation simultanée de deux pompes couplées pourvues de deux tuyaux flexibles mis en parallèle sur lesquels agissent respectivement deux rotors à galets. Un premier type de ce genre de pompe est décrit dans le document WO 82/04 291 où les galets d'un rotor sont décalés angulairement par rapport aux galets du rotor voisin. Un second type de ce genre de pompe est décrit dans le document GB 1 595 901 où les deux rotors présentent des galets coaxiaux deux à deux et où les tuyaux flexibles sont décalés l'un par rapport à l'autre de telle manière que quand un galet du premier rotor écrase le premier tuyau au milieu de sa longueur active, le second tuyau est écrasé simultanément par deux galets du second rotor au début et à la fin de la longueur active dudit second tuyau. Quelle que soit la solution choisie, on comprendra que, de la façon décrite, on obtient un débit beaucoup plus régulier puisque les débits sinusoïdaux présentés par les deux pompes prises indépendamment sont décalés dans le temps et se compensent de façon à obtenir un débit sensiblement constant.

Le document Life Support Systems (1983), 1, 23-28 décrit une pompe péristaltique implantable dans le corps. Cette particularité fait que tous les composants de la pompe sont encapsulés hermétiquement dans un boîtier en titane. Le réservoir de cette pompe est rempli de manière percutanée au moyen d'une seringue. Le moteur d'entraînement de la pompe est du type pas à pas. Il est alimenté par une base de temps suivie d'un diviseur de fréquence et par une batterie. La pompe est programmable extérieurement au moyen d'un appareil de commande relié par induction magnétique à la pompe.

Le document FR-A-2 479 692 (= US-A-4 692 147) décrit également une pompe péristaltique implantable dans le corps dont les caractères génériques répondent en tous points à ceux décrits au paragraphe ci-dessus.

Les pompes péristaltiques connues des documents cités plus haut présentent des principes de constructions qui s'apparentent dans les grandes lignes à la définition donnée au premier paragraphe de cette description qui décrit partiellement la pompe selon la présente invention. Toutefois, aucune des pompes citées et généralement aucune autre pompe connue couramment dans la pratique n'est susceptible d'être portable, par exemple sur le corps humain, pour l'injection lente et continue de préparations médicamenteuses. Les pompes de l'art antérieur sont lourdes et volumineuses et, si elles sont utilisées pour de la perfusion, sont disposées sur une table à proximité d'un malade couché sur un lit.

Au contraire de cela, la pompe selon la présente invention est portable à même le corps, ce qui permet au malade de se déplacer et même de vaquer à ses occupations habituelles. Pour atteindre ce but, la pompe de l'invention est une pompe miniature prenant peu de place. Un autre but que poursuit la pompe selon l'invention est celui d'être bon marché, à tel point qu'elle peut être jetée après usage ce qui prévient tout risque de contamination. Encore un autre but que poursuit la pompe selon l'invention est celui de présenter deux modules - un module pompe et un moteur moteur - séparables par le praticien lui-même qui peut être un médecin ou un infirmier, ceci afin de permettre la stérilisation du module pompe et/ou le choix d'un module moteur adapté à la maladie à soigner.

Pour parvenir à ces buts et aux avantages qu'ils apportent, la pompe péristaltique miniature portable de l'invention est caractérisée par le fait qu'elle comporte un premier module, dit module pompe, et un second module, dit module moteur, lesdits modules étant susceptibles d'être assemblés et désassemblés l'un par rapport à l'autre, le module pompe comprenant la pompe proprement dite munie de son élément de couplage et le module moteur comprenant le moteur pas à pas, le rouage démultiplicateur muni de sa prise de force, le circuit de commande, la base de temps, le diviseur de fréquence et la pile d'alimentation, ledit élément de couplage étant en prise avec ladite prise de force quand lesdits modules sont assemblés.

L'invention sera comprise maintenant à l'aide de la description qui va suivre et qui donne, à titre d'exemple et à l'aide du dessin qui l'accompagne, un mode de réalisation de l'invention, dessin dans lequel :

- la figure 1 est une vue en perspective de la pompe selon l'invention, vue montrant au bas de la figure un module pompe et, au haut, un module moteur, ces deux modules étant désassemblés,

- la figure 2 est une vue en plan du module pompe, le couvercle et certaines autres pièces ayant été enlevés pour faciliter la compréhension,

- la figure 3 est une coupe de la pompe selon l'invention pour laquelle le module pompe a été coupé selon la ligne III-III de la figure 2,

- la figure 4 est une vue en plan partielle du module moteur de la pompe, et

- la figure 5 montre comment peut être portée sur le corps humain la pompe péristaltique miniature selon l'invention.

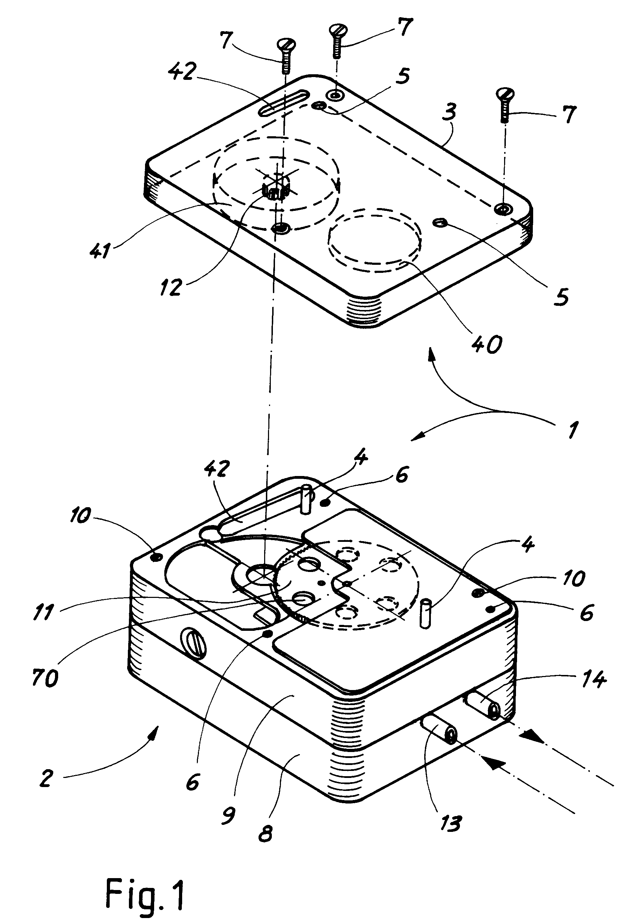

La figure 1 montre une pompe péristaltique 1 exécutée selon un mode de réalisation préféré de l'invention. La pompe comporte un module pompe 2 et un module moteur 3. Comme on le voit sur la figure, les modules 2 et 3 sont susceptibles d'être assemblés et désassemblés l'un par rapport à l'autre. Pour cela le module pompe porte des tiges de centrage 4 qui sont introduites dans des trous de centrage 5 percés dans le module moteur quand les deux modules sont assemblés. Le module pompe porte des taraudages 6 qui reçoivent des vis 7 grâce auxquelles les modules peuvent être fixés l'un à l'autre. Comme le montre encore la figure 1, le module pompe 2 est réalisé en deux parties comprenant un corps de pompe proprement dit 8 et un couvercle 9. Le corps de pompe et le couvercle sont fixés ensemble au moyen de vis 10. Le module pompe 2 est muni d'un élément de couplage, en l'occurrence une roue 11, par lequel la pompe peut être entraînée et le module moteur 3 comporte une prise de force, en l'occurrence un pignon 12, susceptible d'entraîner l'élément de couplage 11 quand les deux modules sont assemblés. Le module pompe 2 comporte encore une entrée 13 et une sortie 14 pour une préparation médicamenteuse à l'état aqueux.

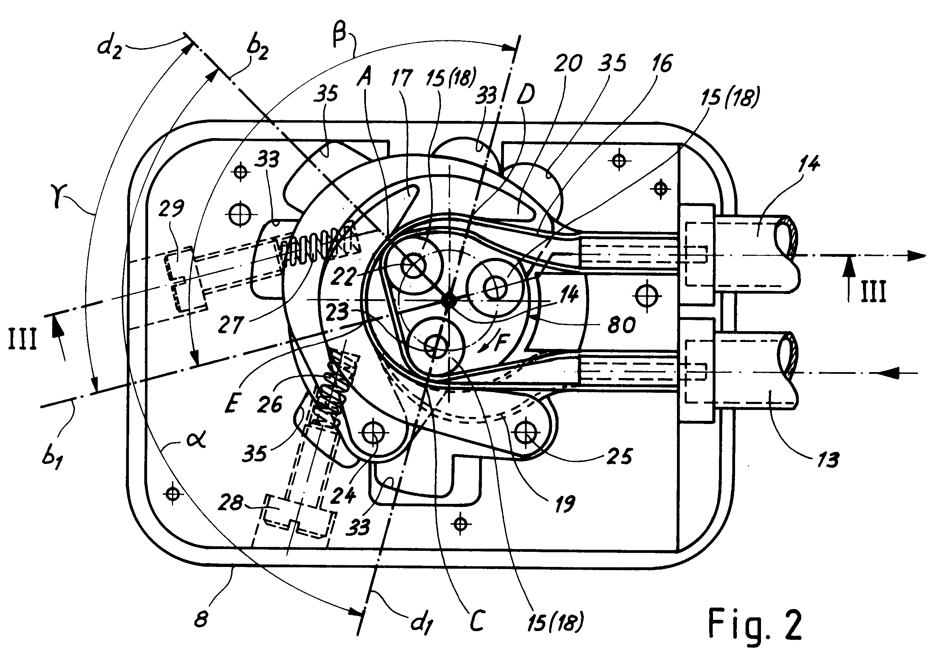

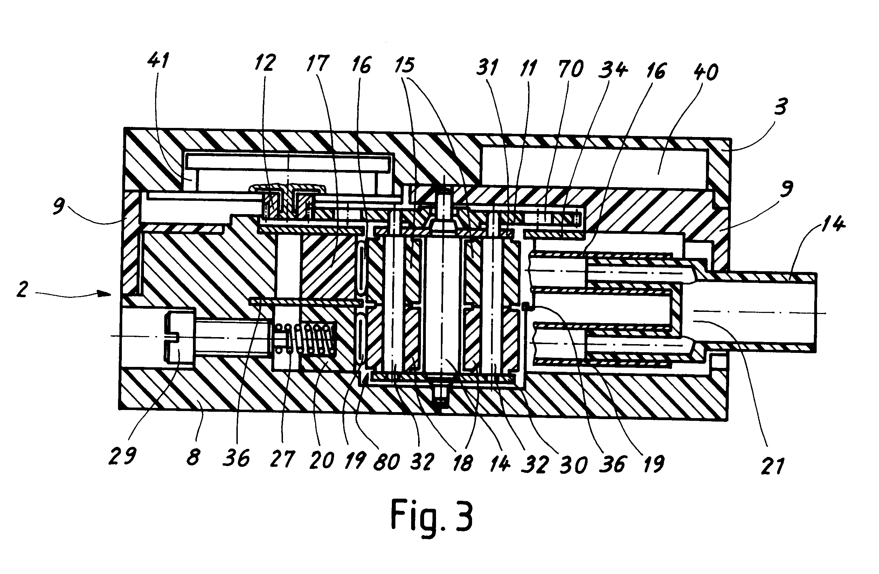

Une exécution préférée du module pompe va être décrite maintenant au moyen des figures 2 et 3. Ce module est équipé d'un rotor 80 portée par un arbre 14. Le rotor 80 comporte un premier jeu de galets 15 roulant le long d'un premier tuyau flexible 16 en le comprimant localement contre un premier presseur 17. Ce premier presseur entoure le premier tuyau selon un premier arc déterminé AC. De façon analogue, le rotor 80 comporte aussi un second jeu de galets 18 roulant le long d'un second tuyau flexible 19 en le comprimant localement contre un second presseur 20. Ce second presseur entoure le second tuyau selon un second arc déterminé DE. Les figures 2 et 3 montrent que les galets 15 et 18 sont disposés coaxialement deux à deux et que les arcs AC et DE présentent sensiblement la même longueur. On voit de même sur la figure 3 que les sorties des tuyaux 16 et 19 sont connectées en parallèle au moyen d'un raccord en Y référencé par 21 pour aboutir à une sortie commune 14, si le rotor tourne dans le sens de la flèche F. Il en est de même pour l'entrée commune 13, bien que la connexion en parallèle n'apparaisse pas à la figure 3. La figure 2 montre en outre que les premier et second presseurs 17 et 20 sont décalés angulairement de telle manière que les bissectrices $b_1$ et $b_2$ des angles $\alpha$ et $\beta$ qu'ils sous-tendent respectivement forment entre elles un angle $\gamma$ qui est la moitié de l'angle (par exemple l'angle $\alpha$) formé par deux droites $d_1$ et $d_2$ issues de l'arbre 14 du rotor et coupant respectivement les axes 22, 23 de deux galets voisins 15.

Cette exécution permet de régulariser le débit de la pompe comme cela a été expliqué plus haut à propos du document GB 1 595 901. A ce propos, on comprendra que si chaque jeu de galets comporte trois galets 15, respectivement 18, également répartis autour de l'arbre 14 du rotor 80, les presseurs 17 et 20 seront décalés d'un angle sensiblement égal à 60°, ce qui d'ailleurs est le cas montré en figure 2.

Les presseurs appuyant les tuyaux contre le rotor peuvent être réalisés de multiples façons. Dans la réalisation de la figure 2, les presseurs 17 et 20 présentent chacun la forme d'un crochet articulé à l'une de ses extrémités, par exemple autour d'une goupille 24, respectivement 25. Les crochets 17 et 20 sont pressés contre les tuyaux 16, respectivement 19 à l'aide de ressorts 26, respectivement 27, eux-mêmes maintenus en place par des vis 28, 29 vissées dans le corps de pompe 8.

Si l'on se réfère à la figure 3, on s'aperçoit que le rotor est formé de deux flasques 30 et 31 entre lesquels sont maintenus les galets 15 et 18 susceptibles de tourner autour d'axes 32. On remarque ici que les galets 15 sont indépendants des galets 18, ce qui leur permet de tourner à des vitesses différentes. On profite des extrémités des axes 32 pour fixer la roue 11 au rotor 80. La figure 3 montre encore que les premier et second presseurs 17 et 20 sont séparés par une plaque 36 qui entoure le rotor 80 et qui est disposée perpendiculairement à l'arbre 14. Cette plaque sert d'entretoise pour les presseurs, ce qui leur confère plus de liberté d'action. La plaque sert également de guide pour chacun des tuyaux 16 et 19 qu'elle maintient au niveau des galets 15 et 18 respectivement. Cette plaque n'est pas dessinée sur la figure 2, afin de ne pas encombrer inutilement ladite figure. On a référencé cependant en figure 2 des logements 33 dans lesquels la plaque 36 prend appui. La figure 3 montre encore une seconde plaque 34 située sous la roue 11 et qui sert également de guide vers le haut pour le tuyau flexible 16. Cette seconde plaque n'est pas non plus représentée sur la figure 2 où, par contre, des logements 35 sont montrés qui servent de points d'appui à la plaque 34.

La description du module pompe qui vient d'être faite est basée sur l'utilisation de deux tuyaux flexibles mis en parallèle et cela pour les motifs indiqués. L'invention n'est cependant pas limitée à une pompe présentant deux tuyaux, celle-ci pouvant n'en présenter qu'un ou plus de deux.

Un mode d'exécution du module moteur va être décrit maintenant au moyen des figures 1, 3 et 4. Selon l'invention, le module moteur comporte un moteur pas à pas entraînant un rouage démultiplicateur portant en fin de chaîne une prise de force figurée par le pignon 12 de la figure 1. Comme on l'a déjà signalé, le pignon 12 est susceptible d'entraîner la roue 11 du module moteur quand les deux modules sont assemblés. Toujours selon l'invention, le moteur pas à pas est alimenté en impulsions d'alimentation par un circuit de commande contrôlé par un diviseur de fréquence recevant un signal en provenance d'une base de temps, le tout étant alimenté par une source de tension continue fournie par une pile. Sur la figure 1, le module moteur montre un logement 40 servant à recevoir la pile et un logement 41 servant à recevoir le rouage, le moteur, le circuit de commande, le diviseur et la base de temps. Une ouverture 42 sert à loger un interrupteur pour mettre le système en marche ou le mettre hors circuit. La figure 3 qui montre une coupe de la pompe complètement assemblée, laisse aussi apparaître les logements 40 et 41 ainsi que la prise de force ou pignon 12 du module moteur en prise avec l'élément de liaison ou roue 11 du module pompe.

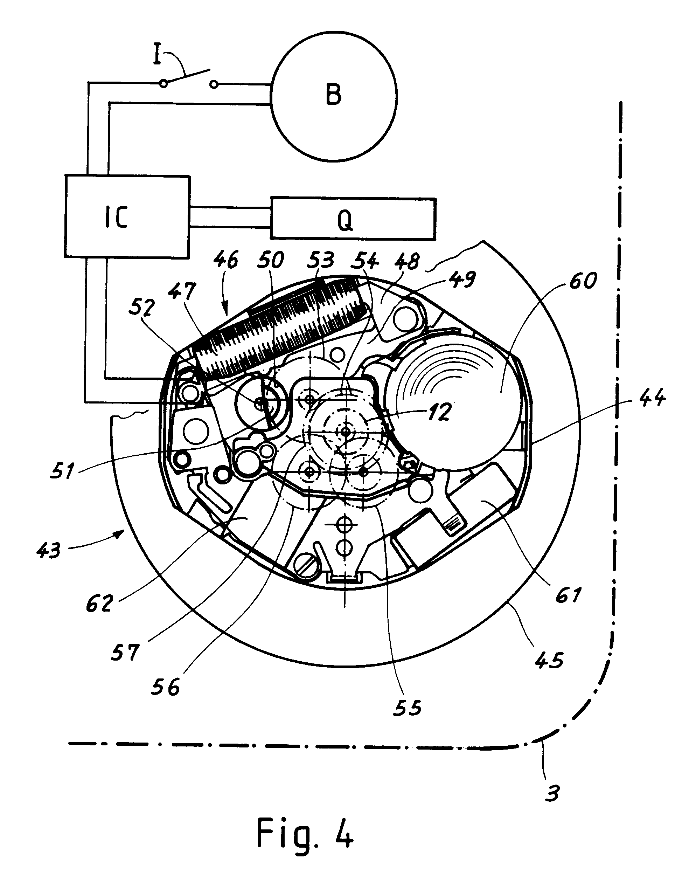

La figure 4, qui est une vue partielle du module moteur de la pompe, montre en détail un mécanisme d'entraînement 43 comportant un mouvement 44 fixé sur une plaque 45, cette dernière étant assujettie au module pompe 3. Le tout prend place dans le logement 41 esquissé en figure 1. Le mouvement 44 comporte un moteur pas à pas 46, de préférence du type de celui utilisé dans une montre-bracelet et dont une description de principe est donnée, par exemple, dans le brevet US-A-2 909 685. La figure 4 montre que le moteur 46 comporte une bobine 47, un noyau 48 et un stator 49 présentant deux pôles séparés par une ouverture circulaire 50 dans laquelle se meut un rotor 51 aimanté diamétralement. Sur l'axe du rotor se trouve un pignon 52 qui entraîne un rouage comportant une suite cinématique de mobiles référencés dans l'ordre d'enchaînement par 53, 54, 55, 56 et 57.

Sur l'axe du mobile 57 se trouve la prise de force ou pignon 12 dont on a déjà parlé. De préférence, tout le mouvement 44 comportant le moteur et le rouage, est un mouvement du type horloger tel qu'utilisé, par exemple, dans une montre-bracelet. Dans ce cas, le mobile 54 est appelé roue des secondes et le mobile 57, roue des heures. Dans cette application particulière, le pignon de sortie 12 remplace l'aiguille des heures.

Comme le montre encore la figure 4, le moteur 46 est alimenté par un bloc IC comprenant un circuit de commande lui-même piloté par un diviseur de fréquence recevant le signal délivré par une base de temps Q. La base de temps sera de préférence un oscillateur à quartz connu pour sa grande stabilité, ce qui confère à la pompe un débit constant. Le bloc IC est communément formé d'un circuit intégré monobloc. Une pile ou batterie B reliée au système par un interrupteur I fournit l'énergie nécessaire. Le système de commande de la marche du moteur est également connu de la technique utilisée depuis longtemps dans les montres-bracelets. On en trouvera une description par exemple dans le brevet US-A-3 742 697.

La figure 4 montre aussi que le mouvement 44 a été débarrassé de la pile, du quartz et du circuit intégré qui normalement trouvent leur place aux endroits référencés par 60, 61 et 62 respectivement. Dans le mode d'exécution pris en exemple, on a préféré disposer des éléments en dehors du mouvement.

On donnera maintenant un exemple de réalisation pratique de l'invention. Le moteur pas à pas choisi fonctionne correctement jusqu'à une fréquence d'environ 105 Hz. En partant d'un quartz horloger classique réglé sur 32'768 Hz, on commence par diviser la fréquence au moyen de six diviseurs par deux, ce qui donne une fréquence de 512 Hz qu'on divise encore une fois par un diviseur par cinq, ce qui aboutit à une fréquence d'alimentation du moteur de 102,4 Hz. Comme le moteur choisi est du type monophasé bipolaire, son arbre fera deux pas par tour et tournera à la vitesse de 51,2 tours par seconde. Entre l'arbre du moteur portant le pignon 52 et l'arbre de la pompe portant la roue 11, la réduction est de 1:129'600, ce qui donne une vitesse de rotation de la roue 11 d'un tour en 42 minutes 11 secondes. Avec cette vitesse de rotation, on a mesuré un débit moyen de 70 mm³ pendant le même laps de temps. Il est bien clair que les chiffres donnés sont un exemple de réalisation et que le débit peut être modifié en agissant sur la fréquence du quartz, sur le nombre d'étages diviseurs, sur la réducton apportée par le train d'engrenages et/ou enfin sur le type de moteur utilisé.

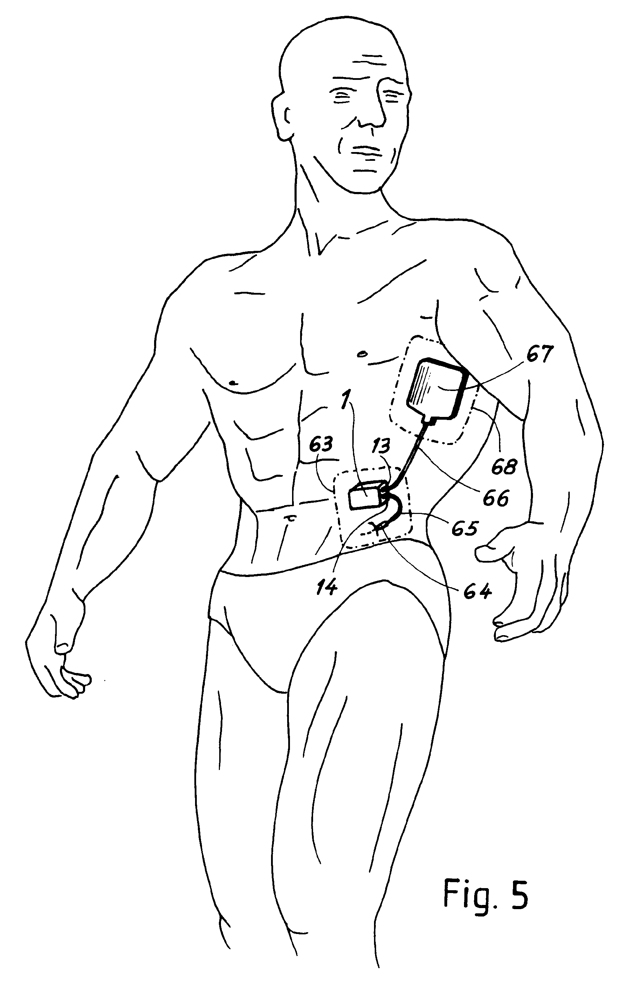

La pompe péristaltique selon l'invention a pour caractéristique principale d'être de très petites dimensions en sorte qu'elle peut être portée directement sur le corps, comme le montre la figure 5. Ici la pompe 1 est posée sur l'abdomen du corps et y est maintenue au moyen d'un adhésif 63. La sortie 14 de la pompe est reliée à une aiguille hypodermique 64 par un conduit 65. L'entrée 13 de la pompe est reliée, au moyen d'un conduit 66 à un réservoir 67 contenant la préparation médicamenteuse. Le réservoir, placé dans la région de l'aisselle, est maintenu sur le corps au moyen d'un autre adhésif 68.

On a vu ci-dessus que le débit de la pompe réalisée selon l'invention dispense un faible débit de liquide, ce qui est une condition pour sa miniaturisation. Elle est ainsi destinée à guérir des maladies autres que le diabète pour lequel de forts débits sont nécessaires à certains instants de la journée. Il n'est en principe pas prévu qu'elle puisse délivrer des débits variables au cours du temps, ce débit pouvant être par contre interrompu si nécessaire pendant certaines périodes grâce à l'interrupteur incorporé. Si les performances de la pompe sont limitées, cette pompe peut cependant être utilisée dans de nombreux cas pour lesquels elle sera appréciée pour sa petitesse et son autonomie. Elle sera aussi appréciée pour son prix très bas, puisqu'elle ne comprend que des composants bon marché et réalisés en grandes séries, particulièrement pour la partie du module moteur. De ce fait, la pompe peut être jetée après emploi.

Il est également important que la pompe soit réalisée en deux modules séparables. On sait en effet qu'il est nécessaire de stériliser la pompe avant emploi, ce qui peut se faire au moyen de radiations gamma. Le module pompe ne portant aucun élément électrique et réalisé presqu'entièrement en matière plastique est bien adapté à ce genre de stérilisation. Le module moteur par contre ne supporterait pas un tel traitement (moteur, circuit intégré, etc.). De plus, si les modules sont séparables, on comprend qu'avec un même module pompe on peut utiliser différents modules moteur, différents surtout par la vitesse de rotation de la prise de force, selon le volume de liquide à injecter.

On a expliqué ci-dessus que la pompe de l'invention présente généralement un faible débit et l'on comprendra que la vitesse de rotation de la roue 11 est si faible qu'elle ne permet pas l'amorçage de la pompe. Pour permettre cette opération, la roue 11 comporte une série de trous 70 (voir figures 1 et 3) qui sont rendus accessibles au praticien avant que le module moteur ne soit monté sur le module pompe. En utilisant une pointe de stylo par exemple, le praticien peut activer la roue 11 à une vitesse permettant l'armorçage de la pompe.

On mentionnera encore que la pompe peut être rendue étanche aux poussières, aux sécrétions naturelles du corps et à l'eau grâce à des moyens d'enrobage enveloppant toute la pompe. Cependant, à la place d'un enrobage, on pourrait, par construction et au moyen de garnitures d'étanchéité, assurer l'étanchéité du corps de pompe 8 par rapport au couvercle 9 et l'étanchéité du module pompe 2 par rapport au module moteur 3. Dans ce dernier cas, on rendra également étanches toutes les ouvertures, telles que les sortie et entrée 13 et 14, la zone 42 où se trouve l'interrupteur 1 et, en général tous les trous de passages (vis, etc.).

On a dit plus haut qu'au moins le module pompe était réalisé en matière plastique. Très généralement, on choisira une matière pour les deux modules qui soit insensible aux attaques des sécrétions naturelles du corps humain.

## Revendications

1. Pompe péristaltique miniature portable (1) notamment sur le corps humain pour l'injection lente et continue de préparations médicamenteuses à l'état acqueux, comportant une pompe équipée d'un rotor (80) porté par un arbre (14) autour duquel sont également répartis des galets (15) animés d'un mouvement de révolution roulant le long d'au moins un tuyau flexible (16) en le comprimant localement contre au moins un presseur (17) entourant ledit tuyau selon un arc déterminé (AC) de manière à permettre l'aspiration et le refoulement de ladite préparation médicamenteuse, ledit arbre étant muni d'un élément de couplage (11), un moteur pas à pas (46) entraînant un rouage démultiplicateur (52 à 57) portant en fin de chaîne une prise de force (12), un circuit de commande pour fournir au moteur des impulsions d'alimentation issues d'une base de temps (Q) suivie d'un diviseur de fréquence et une pile d'alimentation (B), caractérisée par le fait qu'elle comporte un premier module, dit module pompe (2), et un second module, dit module moteur (3), lesdits modules étant susceptibles d'être assemblés et désassemblés l'un par rapport à l'autre, le module pompe (2) comprenant la pompe proprement dite munie de son élément de couplage (11) et le module moteur (3) comprenant le moteur pas à pas (46), le rouage démultiplicateur (52 à 57) muni de sa prise de force (12), le circuit de commande, la base de temps (Q), le diviseur de fréquence et la pile d'alimentation (B), ledit élément de couplage (11) étant en prise avec ladite prise de force (12) quand lesdits modules sont assemblés.

2. Pompe péristaltique selon la revendication 1, caractérisée par le fait que la base de temps est un oscillateur à quartz (Q).

3. Pompe péristaltique selon la revendication 1, caractérisée par le fait qu'au moins le module pompe (2) est contenu dans un boîtier réalisé en matière plastique.

4. Pompe péristaltique selon la revendication 1, caractérisée par le fait que le moteur pas à pas et le rouage démultiplicateur forment un mouvement (44) du type tel qu'utilisé pour l'entraînement des aiguilles d'une montre-bracelet.

5. Pompe péristaltique selon la revendication 1, caractérisée par le fait que l'élément de couplage est une roue dentée (11) et que la prise de force est un pignon denté (12).

6. Pompe péristaltique selon la revendication 5, caractérisée par le fait que la roue dentée (11) est percée d'une pluralité de trous (70) rendus accessibles au praticien quand le module moteur est séparé du module pompe pour permettre l'amorçage de la pompe.

7. Pompe péristaltique selon la revendication 1, caractérisée par le fait que le module pompe comporte des premier (16) et second (19) tuyaux flexibles dont les entrées et les sorties sont connectées respectivement en parallèle et que le rotor (80) comporte un double jeu de galets (15, 18) disposés coaxialement deux à deux, le premier jeu roulant le long du premier tuyau flexible (16) en le comprimant localement contre un premier presseur (17) entourant ledit premier tuyau selon un premier arc déterminé (AC) et le second jeu roulant le long du second tuyau flexible (19) en le comprimant localement contre un second presseur (20) entourant ledit second tuyau selon un second arc déterminée (DE), lesdits premier et second arcs présentant sensiblement la même longueur, lesdits premier et second presseurs étant décalés angulairement de telle manière que les bissectrices ($b_1$, $b_2$) des angles ($\alpha$, $\beta$) qu'ils sous-tendent forment entre elles un angle ($\gamma$) qui est la moitié de l'angle formé par deux droites ($d_1$, $d_2$) issues de l'arbre du rotor et coupant respectivement les axes (22, 23) de deux galets voisins d'un même jeu de galets.

8. Pompe péristaltique selon la revendication 7, caractérisée par le fait que chaque jeu de galets comporte trois galets (15, 18) également répartis autour de l'arbre du rotor et que les premier et second presseurs sont décalés d'un angle ($\gamma$) sensiblement égal à 60°.

9. Pompe péristaltique selon la revendication 7, caractérisée par le fait que les premier (17) et second (20) presseurs présentent chacun la forme d'un crochet articulé à l'une de ses extrémités, ledit crochet étant pressé contre le tuyau correspondant à l'aide d'un ressort (26, 27).

10. Pompe péristaltique selon la revendication 7, caractérisée par le fait que les premier (17) et second (20) presseurs sont séparés par une plaque (36) entourant le rotor et disposés perpendiculairement à son arbre, ladite plaque servant conjointement d'entretoise pour lesdits presseurs et de guide pour lesdits premier (16) et second (19) tuyaux de telle manière à maintenir chaque tuyau au niveau du jeu de galets correspondant.

## Patentansprüche

1. Peristaltische Miniaturpumpe (1), die insbesondere am menschlichen Körper tragbar ist für die langsame und kontinuierliche Injektion von wässrigen medikamentösen Zubereitungen, umfassend eine Pumpe, die mit einem von einer Welle (14) getragenen Rotor (80) versehen ist, um welche Welle herum gleichermaßen verteilt Rollsteine (15) angeordnet sind, die zu einer Umlaufbewegung angetrieben werden, bei der sie längs mindestens eines flexiblen Rohres (16) rollen unter lokaler Komprimierung desselben gegen mindestens ein Druckstück (17), welches das Rohr gemäß einem bestimmten Bogen (AC) umschließt, derart, daß das Ansaugen und Austreiben der medikamentösen Zubereitung ermöglicht wird, welche Welle mit einem Kupplungselement (11) versehen ist, wobei ein Schrittmotor (46) ein Untersetzungsgetriebe (52 bis 57) antreibt, das am Ende der Kette einen Kraftabgriff (12) trägt, und mit einer Steuerschaltung zum Liefern von Speiseimpulsen an den Motor, abgeleitet von einer Zeitbasis (Q), gefolgt von einem Frequenzteiler und einer Speisebatterie (B), dadurch **gekennzeichnet**, daß sie einen ersten als Pumpenmodul (2) bezeichneten Modul umfaßt sowie einen als Motormodul (3) bezeichneten zweiten Modul, welche Moduln miteinander zusammenfügbar und voneinander lösbar sind, wobei der Pumpenmodul (2) die eigentliche Pumpe, versehen mit ihrem Kupplungselement (11), umfaßt und der Motormodul (3) den Schrittmotor (46), das Untersetzungsgetriebe (52 bis 57) mit seinem Kraftabgriff (12), die Steuerschaltung, die Zeitbasis (Q), den Frequenzteiler und die Speisebatterie (B) umfaßt, wobei das Kupplungselement (11) im Eingriff mit dem Kraftabgriff (12) steht, wenn die Moduln zusammengefügt sind.

2. Peristaltische Pumpe nach Anspruch 1, dadurch gekennzeichnet, daß die Zeitbasis ein Quartzoszillator (Q) ist.

3. Peristaltische Pumpe nach Anspruch 1, dadurch gekennzeichnet, daß mindestens der Pumpenmodul (2) in einem aus einem Kunststoffmaterial gefertigten Gehäuse enthalten ist.

4. Peristaltische Pumpe nach Anspruch 1, dadurch gekennzeichnet, daß der Schrittmotor und das Untersetzungsgetriebe ein Werk (44) bilden, der Bauart, wie es für den Antrieb der Zeiger einer Armbanduhr verwendet wird.

5. Peristaltische Pumpe nach Anspruch 1, dadurch gekennzeichnet, daß das Kupplungselement ein Zahnrad (11) ist und der Kraftabgriff ein Ritzel (12) ist.

6. Peristaltische Pumpe nach Anspruch 5, dadurch gekennzeichnet, daß das Zahnrad (11) von einer Mehrzahl von Löchern (70) durchsetzt ist, die für den Praktiker zugänglich sind, wenn der Motormodul von dem Pumpenmodul getrennt ist, um das Starten der Pumpe zu ermöglichen.

7. Peristaltische Pumpe nach Anspruch 1, dadurch gekennzeichnet, daß der Pumpenmodul einen ersten (16) und zweiten (19) flexiblen Schlauch umfaßt, deren Einlässe und Auslässe jeweils parallel geschaltet sind, und daß der Rotor (80) einen Doppelsatz von Rollsteinen (15, 18) umfaßt, die paarweise koaxial angeordnet sind, wobei der erste Satz längs des ersten flexiblen Schlauchs (16) rollt und ihn lokal gegen ein erstes Druckstück (17) komprimiert, das den ersten Schlauch gemäß einem ersten vorbestimmten Bogen (AC) umschließt, und der zweite Satz längs des zweiten flexiblen Schlauchs (19) rollt und ihn lokal gegen ein zweites Druckstück (20) komprimiert, welches den zweiten Schlauch gemäß einem zweiten vorbestimmten Bogen (DE) umschließt, wobei der erste und der zweite Bogen im wesentlichen die gleiche Länge aufweisen, das erste und zweite Druckstück in Winkelrichtung derart versetzt sind, daß die Winkelhalbierenden ($b_1$, $b_2$) der Winkel ($\alpha$, $\beta$), welche sie umschließen, miteinander einen Winkel ($\gamma$) bilden, der die Hälfte des Winkels ist, gebildet von den beiden Geraden ($d_1$, $d_2$), die von der Welle des Motors ausgehen und die Achsen (22, 23) der beiden benachbarten Rollsteine ein und desselben Rollsteinsatzes schneiden.

8. Peristaltische Pumpe nach Anspruch 7, dadurch gekennzeichnet, daß jeder Satz von Rollsteinen drei Rollsteine (15, 18) umfaßt, die gleichförmig verteilt um die Rotorwelle angeordnet sind, und daß das erste und das zweite Druckstück um einen Winkel ($\gamma$) im wesentlichen gleich 60° versetzt sind.

9. Peristaltische Pumpe nach Anspruch 7, dadurch gekennzeichnet, daß das erste (17) und das zweite (20) Druckstück jeweils die Form eines an einem seiner Enden angelenkten Hakens aufweist, welcher Haken gegen den entsprechenden Schlauch mit Hilfe einer Feder (26, 27) angepreßt ist.

10. Peristaltische Pumpe nach Anspruch 7, dadurch gekennzeichnet, daß das erste (17) und zweite (20) Druckstück durch eine Platte (36) getrennt sind, die den Rotor umschließt und senkrecht zu seiner Welle angeordnet ist, welche Platte zugleich als Zwischenstütze für die Druckstücke dient und der Führung des ersten (16) und zweiten (19) Schlauchs derart, daß jeder Schlauch in Höhe des entsprechenden Satzes von Rollsteinen gehalten wird.

## Claims

1. Miniature peristaltic pump (1) especially adapted to be carried on the human body for slow and continuous injection of medicinal preparations in the aqueous state, including a pump having a rotor (80) borne on a shaft (14) around which are evenly distributed rollers (15) given a revolving movement rolling along at least one flexible tube (16) so as to compress it against at least one backing (17) which surrounds said tube over a predetermined arc (AC) so as to permit the sucking in and the expelling of said medicinal preparation, said shaft being provided with a coupling element (11), a stepping motor (46) driving a down gearing chain (52 to 57) bearing at its end a power take-off (12), a control circuit for providing the motor with energizing pulses coming from a time base (Q) followed by a frequency divider and a power cell (B), characterized by the fact that it includes a first or pump module (2) and a second or motor module (3), said modules being adapted to be assembled with or separated from one another, the pump module (2) comprising the pump itself provided with its coupling element (11) and the motor module (3) comprising the stepping motor (46), the down gearing chain (52 to 57) provided with its power take-off (12), the control circuit, the time base (Q), the frequency divider and the power cell (B), said coupling element (11) being engaged with said power take-off (12) when said modules are assembled.

2. Peristaltic pump according to claim 1, characterized by the fact that the time base comprises a quartz oscillator (Q).

3. Peristaltic pump according to claim 1, characterized by the fact that at least the pump module (2) is accommodated in a case formed of plastics material.

4. Peristaltic pump according to claim 1, characterized by the fact that the stepping motor and the down gearing chain form a movement (44) of the type utilized for driving the hands in a wrist watch.

5. Peristaltic pump according to claim 1, characterized by the fact that the coupling element is a toothed wheel (11) and that the power take-off is a toothed pinion (12).

6. Peristaltic pump according to claim 5, characterized by the fact that the toothed wheel (11) is pierced with a plurality of holes (70) accessible to the medical practitioner when the motor module is separated from the pump module so as to permit priming of the pump.

7. Peristaltic pump according to claim 1, characterized by the fact that the pump module includes first (16) and second (19) flexible tubes, the inputs and the outputs of which are respectively connected in parallel and that the rotor (80) includes a double set of rollers (15, 18) coaxially arranged two-by-two, the first set rolling along the first flexible tube (16) and locally compressing it against a first surrounding backing (17) over a first predetermined arc (AC) and the second set rolling along the second flexible tube (19) and locally compressing it against a second surrounding backing (20) over a second predetermined arc (DE), said first and second arcs having substantially the same length, said first and second backings being angularly offset in a manner such that the bisectors ($b_1$, $b_2$) of the angles ($\alpha$, $\beta$) which they subtend form between them an angle ($\gamma$) equal to half the angle formed by two straight lines ($d_1$, $d_2$) issuing from the rotor shaft and cutting respectively the axes (22, 23) of two neighbouring rollers of a same set.

8. Peristaltic pump according to claim 7, characterized by the fact that each set of rollers includes three rollers (15, 18) evenly distributed around the rotor shaft and that the first and second backings are offset by an angle ($\gamma$) substantially equal to 60°.

9. Peristaltic pump according to claim 7, characterized by the fact that the first (17) and second (20) backings each have the form of a hook pivoted at one of its ends, said hook being pressed against the corresponding tube by means of a spring (26, 27).

10. Peristaltic pump according to claim 7, characterized by the fact that the first (17) and second (20) backings are separated by a plate (36) surrounding the rotor and arranged perpendicular to its shaft, said plate serving at the same time as a brace for said backings and as a guide for said first (16) and second (19) tubes so as to maintain each tube at the level of the corresponding set of rollers.

Fig.1

$d_2$  $b_2$  $\beta$

35  $A$  17  15(18)  33  $D$  20  35  16  15(18)  14

$\gamma$

33  29  22  14  80

III  27  23

$b_1$  $E$  26  35  25

$\alpha$  28  24  19  13

8  33  $d_1$  $C$  15(18)

III

10

**Fig. 2**

Fig. 3

EP 0 388 787 B1

I

B

IC

Q

46 50 53 54 48

52 47 49 60

51 12 44

43 62 61

57 45

56 55

Fig. 4

Fig. 5